# EUROPEAN PATENT SPECIFICATION

(11) **EP 3 185 798 B1**
(45) Date of publication and mention of the grant of the patent: **11.03.2020**
(21) Application number: 15836284.8
(22) Date of filing: 25.08.2015
(51) Int. Cl.: A61B 18/04, A61B 18/00, A61B 18/18, A61B 8/12, A61B 8/00, A61B 90/00, A61B 8/08

(54) **SYSTEM FOR PLANNING, MONITORING, AND CONFIRMING TREATMENT**
SYSTEM ZUR PLANUNG, ÜBERWACHUNG UND BESTÄTIGUNG EINER BEHANDLUNG
SYSTÈME POUR LA PLANIFICATION, LA SURVEILLANCE ET LA CONFIRMATION D'UN TRAITEMENT

(30) Priority: 25.08.2014 US 201462041397 P; 21.08.2015 US 201514831983
(43) Date of publication of application: 05.07.2017
(73) Proprietor: Covidien LP, Mansfield, MA 02048 (US)
(72) Inventor: DICKHANS, William J., Longmont, CO 80503 (US); STOPEK, Joshua, Minneapolis, Minnesota 55419 (US); BRANNAN, Joseph D., Lyons, Colorado 80540 (US); LADTKOW, Casey M., Erie, Colorado 80516 (US); PETERSON, Darion R., Boulder, Colorado 80301 (US)
(74) Representative: Maschio, Antonio
(86) International application number: PCT/US2015/046761
(87) International publication number: WO 2016/033090

(56) References cited:
- EP-A1- 2 364 660
- WO-A1-2005/115235
- WO-A1-2008/002517
- WO-A1-2011/104664
- WO-A1-2014/025550
- WO-A2-2009/150563
- WO-A2-2013/158392
- US-A1- 2013 281 851
- US-A1- 2014 046 174
- US-A1- 2014 187 949
- LUO XIONGBIAO ET AL: "Beyond Current Guided Bronchoscopy: A Robust and Real-Time Bronchoscopic Ultrasound Navigation System", 22 September 2013 (2013-09-22), ECCV 2016 CONFERENCE; [LECTURE NOTES IN COMPUTER SCIENCE; LECT.NOTES COMPUTER], SPRINGER INTERNATIONAL PUBLISHING, CHAM, PAGE(S) 388 - 395, XP047041936, ISSN: 0302-9743 ISBN: 978-3-642-33485-6 * the whole document *
- YEHUDA SCHWARZ: "Electromagnetic Navigation", CLINICS IN CHEST MEDICINE, vol. 31, no. 1, 1 March 2010 (2010-03-01), pages 65-73, XP055131118, ISSN: 0272-5231, DOI: 10.1016/j.ccm.2009.08.005

## Description

### BACKGROUND

### Technical Field

The present disclosure relates to a system, apparatus, and method of navigation and position confirmation and treatment zone planning, monitoring, and confirmation for surgical procedures. More particularly, the present disclosure relates to a system and method for enhanced navigation of an extended working channel or catheter and one or more tools positionable therethrough in one or more branched luminal networks of a patient, confirming placement of those tools relative to a target tissue prior to initiating treatment, generating treatment plans for target tissue, and monitoring and confirming the zone treated by the surgical tool after treatment is initiated.

### Description of Related Art

Microwave ablation is a commonly applied method for treating various maladies affecting organs including the liver, brain, heart, lung and kidney. Commonly, one or more imaging modalities, whether magnetic resonance imaging, ultrasound imaging, computer tomography (CT), as well as others will be employed by a clinician to identify areas of interest within a patent and ultimately targets for treatment. Once identified, an area of interest will typically require a biopsy using a biopsy tool to confirm whether treatment or observation is necessitated at a particular time. This biopsy is typically performed under one of a number of image guidance modalities, and/or in conjunction with a navigation system. If the biopsy reveals that the area of interest is malignant, it may prove useful to treat the area using microwave ablation.

Microwave ablation may be performed by transmitting microwave energy through a needle inserted percutaneously in the patient to ablate the area of interest. Alternatively, where practicable, an endoscopic approach can be undertaken, where, once navigated to the identified target, a flexible microwave ablation catheter can be placed in the target to ablate the area of interest. The endoscopic approach is particularly useful when treating luminal networks of the body such as the lungs.

To enable the endoscopic approach, for example in the lungs, endobronchial navigation systems have been developed that use CT image data to create a navigation plan to facilitate advancing a navigation catheter (or other suitable device) through a bronchoscope and a branch of the bronchus of a patient to the area of interest. Endobronchial navigation may be employed both in the diagnostic (i.e., biopsy phase) and the treatment phases. Electromagnetic tracking may be utilized in conjunction with the CT data to facilitate guiding the navigation catheter through the branch of the bronchus to the area of interest. In certain instances, the navigation catheter may be positioned within one of the airways of the branched luminal networks adjacent to or within the area of interest to provide access for one or more tools.

Once the navigation catheter is in position, fluoroscopy may be used to visualize biopsy tools, such as, for example, brushes, needles and forceps, as well as treatment tools such as an ablation catheter, as they are passed through the navigation catheter and into the lung and to the area of interest. Although medical instruments like catheters, biopsy tools, etc., are clearly visible on a fluoroscopic picture, organic features such as soft tissue, blood vessels, suspicious tumor lesions etc., are quite transparent and hard to identify with conventional fluoroscopy. Additionally, fluoroscopic images merely provide a flat 2D image, on which it can be somewhat challenging to assess the depth of the surgical tool relative to a target within an area of interest. As such, the clinician is not provided all the information that could be desired to visualize the placement of the surgical device within the patient's body relative to the area of interest. Further, as organic features such as tissue are difficult to identify in fluoroscopic images, the use of fluoroscopes alone are not ideal for monitoring treatment zones. US2014046174 represents an example of the prior art.

Moreover, using the conventional approach, a clinician is required to perform a biopsy on a target to determine whether treatment of the target is required (diagnostic phase) and only after the diagnosis is made, can make a determination as to whether treatment is necessary (treatment phase). In certain cases, this necessitates multiple navigation procedures and results in longer durations between testing (diagnosis) and actual treatment if treatment is required.

Accordingly, a need exists for confirming placement of a surgical tool relative to a target within a region of interest to test the target and determine whether the target requires treatment, determine a treatment plan, and monitor the treatment of the target before, during, and after treatment of the target to confirm that the entire target has been properly treated.

### SUMMARY

As can be appreciated, a microwave ablation catheter that is positionable through one or more branched luminal networks of a patient to treat tissue may prove useful in the surgical arena.

Aspects of the present disclosure are described in detail with reference to the figures wherein like reference numerals identify similar or identical elements. As used herein, the term "distal" refers to the portion that is being described which is further from a user, while the term "proximal" refers to the portion that is being described which is closer to a user.

According to one aspect of the present disclosure, a system for monitoring a surgical procedure is provided. The system includes a catheter guidance assembly including an extended working channel navigable to a target, a surgical device positionable through the extended working channel to access the target, at least one sensor disposed on either the extended working channel or the surgical device. The at least one sensor is configured to sense a characteristic of tissue proximate either or both of the extended working channel or the surgical device. The system further includes a computing device operably coupled to the sensor. The computing device is configured to receive the characteristic from the sensor and determine whether the tissue characteristic is indicative of the target or tissue other than the target.

In an embodiment, the extended working channel includes an ultrasound transducer configured to generate ultrasound waves and an ultrasound sensor configured to receive ultrasound waves reflected from tissue. The surgical device may be a microwave ablation device configured to deliver microwave energy generated by a microwave generator to the target. The computing device may be configured to display a model of a luminal network and process the sensed ultrasound waves and integrate the sensed ultrasound waves with the model of the luminal network. The computing device may also be configured to identify tissue density based on the characteristic of the tissue, display a treatment zone, and determine whether the entire target has been treated or whether additional application of energy is necessary.

In an embodiment, the sensor is a light receptor configured to sense light reflected from tissue proximate the surgical device. The computing device may convert the reflected light into light based data and identify a type or density of tissue, identify the presence of one or more blood vessels, generate visible images, or integrate the light based data with a model for display. Additionally, or alternatively, the sensor may be a hydration sensor configured to detect water levels of tissue proximate the at least one hydration sensor.

Additionally, or alternatively, the system may further include a radiometer coupled to the sensor. The radiometer is configured to measure emissions from a thermal field created when the surgical device delivers microwave energy, the thermal field providing in-situ quantitative information of a material in the thermal field. The radiometer may be disposed within a handle of the surgical device, or alternatively, external to the surgical device. The quantitative information is at least one of thermal conductivity, specific heat, density, or blood perfusion. The quantitative information is displayed on a display of the computing device.

In another aspect of the present disclosure, a method for monitoring a surgical procedure is provided which includes navigating an extended working channel to a target using a previously planned navigation path, inserting the surgical device into the extended working channel to access the target, applying a non-therapeutic energy to tissue proximate the surgical device, sensing a response of the applied non-therapeutic amount of energy via at least one sensor, determining whether the tissue proximate the surgical device is the target by analyzing the sensed response, generating a treatment plan when it is determined that the tissue proximate the surgical device is the target, applying a therapeutic energy in accordance with the treatment plan, and monitoring the application of the applied therapeutic energy.

In embodiments, the surgical device is a microwave ablation device and the applying a therapeutic energy when it is determined that the tissue proximate the surgical device is the target includes applying microwave energy. The method may further include displaying a representation of an ablation zone created by the therapeutic energy on a graphical user interface. Additionally, or alternatively, the method may further include determining whether the ablation zone exceeds a threshold value, and applying additional therapeutic energy when it is determined that the ablation zone does not exceed the threshold value. The method may further include combining at least a portion of the sensed response with a representation of a lung into a combined image, and displaying the combined image on a graphical user interface. Additionally, the method may further include utilizing the sensor signals or feedback to determine an appropriate ablation dose including a determination of the amount of power to apply and an amount of time to apply the power.

In yet another aspect of the present disclosure, a non-transitory computer readable storage medium is provided. The non-transitory computer readable storage medium includes instructions that, when executed, cause a computing device to display preplanned navigation plan enabling navigation of an extended working channel and a surgical device to a target, sense a response of an applied non-therapeutic amount of energy via a sensor, determine whether the tissue proximate the surgical device is the target by analyzing the sensed response, enable application of a therapeutic energy when it is determined that the tissue proximate the surgical device is the target, and monitor the application of the applied therapeutic energy.

### BRIEF DESCRIPTION OF THE DRAWINGS

Various aspects and embodiments of the present disclosure are described hereinbelow with references to the drawings, wherein:
Fig. 1 is a perspective view of one illustrative embodiment of an electromagnetic navigation (EMN) system in accordance with the present disclosure;
Fig. 2 is a perspective view of one illustrative embodiment of a microwave ablation device and external radiometer usable with the system of Fig. 1 in accordance with the present disclosure;
Fig. 3 is a perspective view of one illustrative embodiment of a microwave ablation device and an internal radiometer usable with the system of Fig. 1 in accordance with the present disclosure;
Fig. 4 is a perspective view of one illustrative embodiment of an extended working channel including an ultrasound transducer usable with the system of Fig. 1 in accordance with the present disclosure;
Fig. 5 is a view of an extended working channel and a microwave ablation device navigated to a region of interest within a bronchial airway of a patient in accordance with the present disclosure;
Fig. 6 is a flow chart of a method for adjusting the position of a surgical device relative to a target in accordance with the instant disclosure; and
Fig. 7 is a flow chart of a method for monitoring an ablation zone in accordance with the present disclosure.

### DETAILED DESCRIPTION

The present disclosure is generally directed to addressing the diagnosis, navigational, and location and monitoring confirmatory shortcomings of the previously known diagnosis, navigation, planning, and confirmation and monitoring methods and devices. According to one embodiment of the present disclosure, following navigation of a catheter to an area of interest, a diagnosis of a target may be performed to determine whether treatment of target tissue is required. The real time diagnosis of tissue serves the dual function of providing details as to the type of tissue (such as either healthy tissue or a lesion requiring treatment) and providing a confirmation of the placement of the surgical device relative to the tissue after a surgical device is navigated to the target. Additionally, the details of the tissue may be utilized to develop or determine a treatment plan including the amount of time to apply energy and the type of energy to apply, or other ablation/treatment levels or doses. Further, using the methodologies described below, a user can monitor and confirm treatment of the tissue as the tissue is being treated and ultimately view the entire zone treated after treatment is initiated.

Detailed embodiments of the present disclosure are disclosed herein. However, the disclosed embodiments are merely examples of the disclosure, which may be embodied in various forms and aspects. Therefore, specific structural and functional details disclosed herein are not to be interpreted as limiting, but merely as a basis for the claims and as a representative basis for teaching one skilled in the art to variously employ the present disclosure in virtually any appropriately detailed structure.

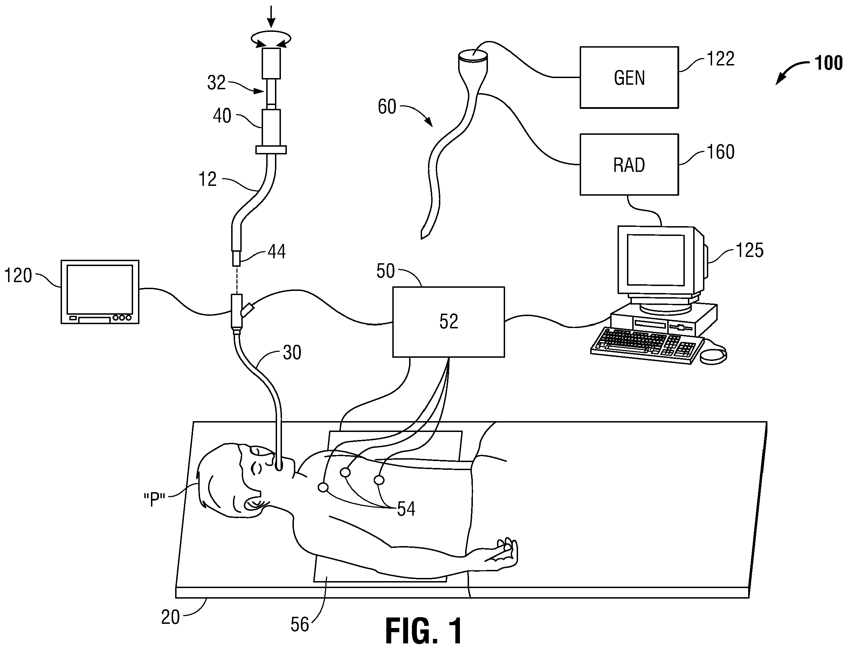

Fig. 1 depicts an aspect of an electromagnetic navigation (EMN) system 100 configured for reviewing CT image data to identify one or more targets 14, planning a pathway to an identified target 14 (planning phase), navigating an extended working channel 12 to the target 14 (navigation phase), and confirming placement of the extended working channel 12 within the target 14. One such ENM system is the ELECTROMAGNETIC NAVIGATION BRONCHOSCOPY® system currently sold by Covidien LP. Following navigation, a surgical tool, such as a microwave ablation device 60, may be inserted into the extended working channel 12 to ablate the target 14.

As shown in Fig. 1, extended working channel 12 is part of a catheter guide assembly 40. In practice, the extended working channel 12 is inserted into bronchoscope 30 for access to a luminal network of the patient "P." Specifically, extended working channel (EWC) 12 of catheter guide assembly 40 may be inserted into a working channel of bronchoscope 30 for navigation through a patient's luminal network. A locatable guide (LG) 32, including a sensor 44 is inserted into the extended working channel 12 and locked into position such that the sensor 44 extends a desired distance beyond the distal tip of the extended working channel 12. The position and orientation (6 DOF) of the sensor 44 relative to the reference coordinate system, and thus the distal end of the extended working channel 12, within an electromagnetic field can be derived. Catheter guide assemblies 40 are currently marketed and sold by Covidien LP under the name SUPERDIMENSION® Procedure Kits, or EDGE™ Procedure Kits, and are contemplated as useable with the present disclosure. For a more detailed description of the catheter guide assemblies 40, reference is made to commonly-owned U.S. Patent Application Serial No. 13/836,203 filed on March 15, 2013 by Ladtkow et al, and U.S. Patent No. 7,233,820.

System 100 generally includes an operating table 20 configured to support a patient "P;" a bronchoscope 30 configured for insertion through the patient's "P's" mouth into the patient's "P's" airways; monitoring equipment 120 coupled to bronchoscope 30 (e.g., a video display, for displaying the video images received from the video imaging system of bronchoscope 30); a tracking system 50 including a tracking module 52, a plurality of reference sensors 54, and a transmitter mat 56; and a computing device 125 including software and/or hardware used to facilitate pathway planning, identification of target tissue, navigation to target tissue, confirmation of placement of an extended working channel 12, or a suitable device therethrough, relative to a target 14, and monitoring the application of microwave energy into a target 14.

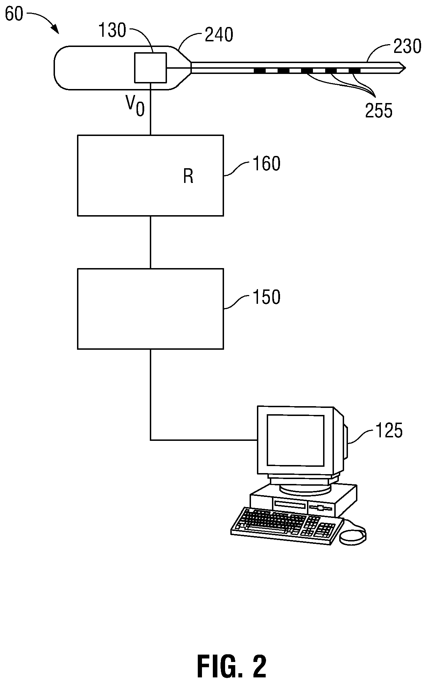

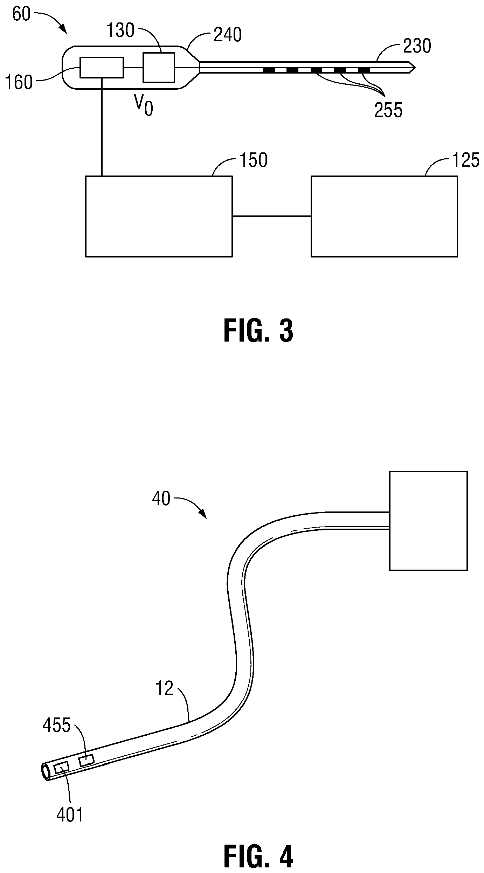

Continuing with reference to Fig. 1, system 100 further includes a microwave ablation device 60 insertable into the extended working channel 12 to access a target 14. The microwave ablation device 60 is coupled to a microwave generator 122. In one embodiment, the microwave ablation device 60 is also coupled to radiometer 160 as will be described in greater detail below. Although radiometer 160 is illustrated as being a separate component from microwave ablation device 60, in embodiments, radiometer 160 may be incorporated into microwave ablation device 60 as a single component of system 100 (as illustrated in Fig. 3). Microwave generator 122 is configured to supply microwave energy to the microwave ablation device 60 to ablate a target 14. Further details regarding the microwave ablation device 60 will be described in greater detail below with reference to Figs. 2 and 3.

Computing device 125 may be any suitable computing device including a processor and storage medium, wherein the processor is capable of executing instructions stored on the storage medium. The computing device 125 may further include a database configured to store patient data, CT data sets including CT images, navigation plans, and any other such data. Although not explicitly illustrated, the computing device 125 may include inputs, or may otherwise be configured to receive, CT data sets and other data described herein. Additionally, computing device 125 includes a display configured to display graphical user interfaces such as those described below. Computing device 125 may be connected to one or more networks through which one or more databases may be accessed.

With respect to the planning phase, computing device 125 utilizes computed tomographic (CT) image data for generating and viewing a three-dimensional model of the patient's "P's" airways, enables the identification of a target 14 on the three-dimensional model (automatically, semi-automatically, or manually), and allows for determining a pathway through the patient's "P's" airways to the target 14. More specifically, the CT scans are processed and assembled into a three-dimensional CT volume, which is then utilized to generate a three-dimensional model of the patient's "P's" airways. The three-dimensional model may be displayed on a display associated with computing device 125, or in any other suitable fashion. Using computing device 125, various views of the three-dimensional model or two-dimensional images generated from the three-dimensional model are presented. The three-dimensional model may be manipulated to facilitate identification of target 14 on the three-dimensional model or two-dimensional images, and selection of a suitable pathway through the patient's "P's" airways to access the target 14 can be made. Once selected, the pathway plan, 3D model, and images derived therefrom can be saved and exported to a navigation system for use during the navigation phase(s). One such planning software is the ILOGIC® planning suite currently sold by Covidien LP.

With respect to the navigation phase, a six degrees-of-freedom electromagnetic tracking system 50, e.g., similar to those disclosed in U.S. Patent Nos. 8,467,589, 6,188,355, and published PCT Application Nos. WO 00/10456 and WO 01/67035, or other suitable positioning measuring system, is utilized for performing registration of the images and the pathway and navigation, although other configurations are also contemplated. Tracking system 50 includes a tracking module 52, a plurality of reference sensors 54, and a transmitter mat 56. Tracking system 50 is configured for use with a locatable guide 32 and particularly sensor 44. As described above, locatable guide 32 and sensor 44 are configured for insertion through an extended working channel 12 into a patient's "P's" airways (either with or without bronchoscope 30) and are selectively lockable relative to one another via a locking mechanism.

As shown in Fig. 1, transmitter mat 56 is positioned beneath patient "P." Transmitter mat 56 generates an electromagnetic field around at least a portion of the patient "P" within which the position of a plurality of reference sensors 54 and the sensor element 44 can be determined with use of a tracking module 52. One or more of reference sensors 54 are attached to the chest of the patient "P." The six degrees of freedom coordinates of reference sensors 54 are sent to computing device 125 (which includes the appropriate software) where they are used to calculate a patient coordinate frame of reference. Registration, as detailed below, is generally performed to coordinate locations of the three-dimensional model and two-dimensional images from the planning phase with the patient's "P's" airways as observed through the bronchoscope 30, and allow for the navigation phase to be undertaken with precise knowledge of the location of the sensor 44, even in portions of the airway where the bronchoscope 30 cannot reach. Further details of such a registration technique and their implementation in luminal navigation can be found in U.S. Patent Application Pub. No. 2011/0085720, although other suitable techniques are also contemplated.

Registration of the patient "P's" location on the transmitter mat 56 is performed by moving LG 32 through the airways of the patient "P." More specifically, data pertaining to locations of sensor element 44, while locatable guide 32 is moving through the airways, is recorded using transmitter mat 56, reference sensors 54, and tracking module 52. A shape resulting from this location data is compared to an interior geometry of passages of the three-dimensional model generated in the planning phase, and a location correlation between the shape and the three-dimensional model based on the comparison is determined, e.g., utilizing the software on computing device 125. In addition, the software identifies non-tissue space (e.g., air filled cavities) in the three-dimensional model. The software aligns, or registers, an image representing a location of sensor 44 with a the three-dimensional model and two-dimensional images generated from the three-dimension model, which are based on the recorded location data and an assumption that locatable guide 32 remains located in non-tissue space in the patient's "P's" airways. Alternatively, a manual registration technique may be employed by navigating the bronchoscope 30 with the sensor 44 to pre-specified locations in the lungs of the patient "P", and manually correlating the images from the bronchoscope to the model data of the 3D model.

Following registration of the patient "P" to the image data and pathway plan, a user interface is displayed in the navigation software which sets for the pathway that the clinician is to follow to reach the target 14. One such navigation software is the ILOGIC® navigation suite currently sold by Covidien LP.

Once extended working channel 12 has been successfully navigated proximate the target 14, the locatable guide 32 may be unlocked from extended working channel 12 and removed, leaving extended working channel 12 in place as a guide channel for guiding surgical instruments including without limitation, optical systems, ultrasound probes, biopsy tools, ablation tools (i.e., microwave ablation device 60), laser probes, cryogenic probes, sensor probes, and aspirating needles to the target 14.

Turning now to Fig. 2, one illustrative embodiment of a microwave ablation device 60 is shown including at least one microwave antenna 230 which emits microwave radiation generated by the generator 122 (Fig. 1) and sensors 255. The microwave antenna 230 is configured to be inserted into the target 14 so that the microwave radiation emitted from the antenna 230 can ablate the target 14 and sense characteristics of surrounding tissue, via sensors 255, as will be described in greater detail below. The microwave ablation device 60 includes a handle 240 coupled to the microwave antenna 230 to allow a clinician to manipulate the microwave antenna 230 during a microwave ablation procedure.

In the embodiment illustrated in Fig. 2, the radiometer 160 is disposed external to the microwave ablation device 60 as a separate component. Effectively, this isolates the radiometer 160 from at least some of the noise of the microwave ablation device 60, which may be desired in some applications. For example, as shown in Fig. 2, the radiometer 160 is disposed between the microwave ablation device 60 and the controller 150.

Fig. 3 depicts another aspect of microwave ablation device 60 where the radiometer 160 is disposed within the handle 240 of the microwave ablation device 60. The microwave ablation device 60 depicted in Fig. 3 is similar to the microwave ablation device 60 illustrated in Fig. 2, and described above, and therefore will not be described in detail. In certain applications, in order to obtain accurate temperature measurements, the radiometer 160 is disposed as close as possible to the radiating portion of the microwave antenna 230 to limit unwanted noise (e.g., from heating of a coaxial cable employed in most microwave antennas) from entering the radiometer 160. For example, as shown in Fig. 3, the radiometer 160 and the coupling circuit 130 are disposed within the handle 240 of the microwave ablation device 60. The coupling circuit 130 is coupled between a microwave feed transmission line and the antenna element to provide at least a portion of a microwave signal propagating in the antenna element to the radiometer 160. The radiometer 160 depicted in Fig. 3 is coupled to the coupling circuit 130 and outputs a voltage signal V₀ or a digital signal derived from the temperature of the environment surrounding the microwave antenna 230, e.g., tissue that is being ablated. That is, the environment having been heated by the microwave antenna 230 emits a noise temperature signal at a particular frequency, and the temperature of the environment may be derived from the intensity of that signal. This voltage signal, V₀, or digital signal is provided to the computing device 125 for processing.

The microwave ablation device 60 acts as both a transmitter and receiver. The microwave antenna 230 may be embodied as an inflexible ablation catheter or a flexible ablation catheter to accommodate a specific surgical procedure, a specific luminal structure, specific target tissue, a clinician's preference, etc. For example, in one embodiment, it may prove advantageous to have an ablation catheter that is very flexible for movement through the relatively narrow airways of the lungs of a patient. In some cases, it may prove advantageous to have an ablation catheter that is only slightly flexible, e.g., where the ablation catheter is needed to pierce or puncture tissue. It should be understood to those of skill in the art that the microwave antenna 230 may employ other ablation catheter embodiments, either simplified or more complex in structural detail, without departing from the scope of the instant disclosure.

The microwave ablation device 60 may be used to sense particular characteristics of tissue. All materials, such as tissue, emit some sort of radiation and different types of tissue emit different radiation. For example, healthy tissue emits a different radiation than tumorous tissue (such as lung lesions). When the material, such as tissue, is heated, the change in temperature results in an increase in the intensity or frequency of those emissions. These emissions are one type of radiation that can be received by the microwave antenna 230 which can provide indications of quantitative information of the material in question. The quantitative information may relate to at least thermal conductivity, specific heat, tissue density, and local perfusion of tissue. The quantitative information may be used to determine the position of the microwave antenna 230 relative to the target. For example, the quantitative information may be used to determine if the microwave antenna 230 is positioned proximate healthy lung tissue or if the microwave antenna is positioned proximate a lung lesion. Such position data of the microwave antenna 230 may be displayed on a display of computing device 125. Additionally, such position data of the microwave antenna 230 may be used as a confirmation for the clinician prior to initiating the application of microwave energy to ablate the target 14. Moreover, the quantitative information may also be used to monitor the ablation zone before, during, and after the application of microwave energy to the target 14 to confirm ablation of the entire target 14. Additionally or alternatively, the sensed signals/quantitative information may also be used to determine a treatment plan including the amount of time to apply energy and the type of energy to apply, or other ablation/treatment levels or doses.

Regarding the quantitative information, the thermal conductivity of biological tissue is dependent on the particular type of biological tissue and on the composition of the biological tissue. Different biological tissues exhibit different and/or unique thermal conductivity based on factors such as tissue density, vascularization, age, direction and distance to major blood vessels, etc. Additionally, different biological tissues may exhibit a different and/or unique thermal conductivity in different directions. Electrical conductivity is not only determined by tissue type and composition, but also by other externally applied physical and chemical influences during thermal treatment, such as, for example, temperature inducement and saline pretreatment. Knowing such characteristics of tissue allow for the creation of and quantification of one or more thermal fields in tissue before and following the application of energy to the tissue.

Therefore, thermal zones based on these emissions of the tissue prior to and/or following application of energy may be quantified and displayed on a display screen of computing device 125, thus allowing a surgeon to create and visualize the thermal environment surrounding a microwave antenna 230 before, during, and after a surgical procedure. Stated differently, the thermal environment may be assessed, in real-time, to allow the surgeon to instantaneously receive feedback, and use that feedback to evaluate different approaches to use during the surgical procedure. Therefore, application of thermal therapy can be continuously adjusted or modified or altered based on real-time feedback related to the thermal zones of tissue that form a thermal environment.

Moreover, multiple thermal environments may be simultaneously assessed during a single surgical procedure. In other words, a surgeon may assess a thermal environment of a plurality of different tissues or organs and compare such thermal environments to determine appropriate action during the surgical procedure. Therefore, the real-time assessment of a plurality of thermal environments influences a surgeon's decision on, for example, how much energy to apply to each of the plurality of tissues. In other words, a direct relationship is established between one or more computed thermal environments of tissue and subsequent energy application to such tissue. Stated differently, thermal characteristics of tissue are used to create one or more thermal fields that form a thermal environment that is displayed to allow for real-time, instantaneous, and continuous adjustments to be made to the computed thermal environments. Such adjustments may relate to, for instance, power adjustments or time-setting adjustments. Of course, one skilled in the art may contemplate making any type of variable or parameter adjustments, continuously and in real-time, to the computed thermal fields.

With reference to Fig. 2, a coupling circuit 130 couples the microwave ablation device 60 to radiometer 160. The coupling circuit 130 receives noise temperature signals or at least a portion of the signals propagating through the microwave cables, and directs the noise temperature signals to the radiometer 160, including those which are received by the microwave ablation device 60 from tissue being treated, such as a tumor or lesion. A filter (not shown) may also be included to isolate the noise temperature signals from the microwave signal generated by the microwave generator 122, including any reflected power resulting from an imbalance between the source and the load impedance. The radiometer 160 samples the noise temperature signals and provides them to a controller 150. The controller 150 converts the microwave noise temperature signal into a temperature reading by digitally sampling the microwave noise temperature signal using an analog-to-digital converter (ADC) and scaling the result. The controller 150 then transmits the data, such as the temperature readings, to the computing device 125 for further processing and display. The computing device 125 may process the data to Additionally or alternatively, the sensed signals may also be used to develop a treatment plan including the amount of time to apply energy and the type of energy to apply, or other ablation/treatment levels or doses.

The coupling circuit 130 is coupled between a microwave feed transmission line and the microwave antenna 230 to provide at least a portion of a microwave signal propagating in the microwave antenna 230 to the radiometer 160. The radiometer 160 is coupled to the coupling circuit 130. The radiometer may output a voltage signal V₀ or a digital signal derived from the temperature of the environment surrounding the microwave antenna 230, e.g., tissue that is being ablated. That is, the environment having been heated by the microwave antenna 230 emits a noise temperature signal at a particular frequency, and the temperature of the environment may be derived from the intensity of that signal. This voltage signal, V₀, or digital signal is provided to the computing device 125 for processing and display.

Continuing with reference to Fig. 2, as described above, microwave ablation device 60 may further include one or more sensors 255 disposed along the length of the microwave antenna 230. Of course, the microwave ablation device 60 of the present disclosure may include any number of sensors 255 incorporated on any portion of the microwave ablation device 60. The sensors 255 may be thermocouples connected to the controller 150 to provide additional temperature data of the microwave antenna 230, cooling channels (not shown) within the cooling channel, or of specific components, such as a balun or choke formed within the microwave antenna 230.

Additionally, or alternatively, sensors 255 may be one more chemical sensors configured to detect one or more chemicals of tissue prior to, during, or after activation of the microwave ablation device 60. In this embodiment, the chemical sensor may be in operable communication with the controller 160 that is configured to detect chemicals associated with the target tissue, e.g., acids and proteins. The chemicals detected may be correlated to a progression of thermal ablation growth and stored in one or more data look-up tables (not shown) that is accessible to the controller 160.

Additionally, or alternatively, as dielectric properties can be directly correlated with hydration levels of the tissue, sensors 255 may be one or more hydration sensors configured to measure the water content of the tissue proximate the microwave antenna 230 to confirm placement of the microwave ablation catheter 60, develop a treatment plan, and monitor ablation progress and/or completion of an ablation procedure. As moisture is driven out of the tissue, the hydration sensors track the rate of change and communicate such data to the controller 160 to ultimately inform the clinician of the status of the ablation, notify the clinician when the ablation of the target 14 is complete, and/or develop a new treatment plan to use.

Additionally, or alternatively, sensors 255 may be one or more light sensors. In an aspect of the present disclosure, fluorescence may be used to identify a target with the spectroscopy using visible light. When a target is identified by the CT or MRI scanning, the target may be dyed by using either fluorescent or auto fluorescent dye. In the case of fluorescent dyes, a light source may be used to emit light to a fluorescently dyed lesion, the fluorescent dye radiates a specific frequency response that can be received or otherwise sensed by the sensors 255 and detected as different from the response of other un-dyed lung tissue. Similarly, but without the need of a light source, auto fluorescent dyes constantly emit a signal at a specific frequency, and these emissions can be received and detected by the sensors 255 as well.

In both cases, the response can be processed and displayed on the display of the computing device 125, thus permitting visualization of the target without requiring spectroscopic analysis to confirm placement of the microwave ablation device 60 relative to the target 14, develop a treatment plan, and to monitor the amount that the target 14 has been ablated or treated. Specifically, after initiating application of the microwave energy, the fluorescence color may change along the treatment. In other words, when the treatment begins, the fluorescent color disappears or changes to a predetermined color in areas that have been impacted by the ablation zone. Thus, by inspecting the fluorescent color of the target 14, a clinician may determine whether the treatment of the target 14 is complete, whether a new treatment plan should be developed, and/or whether additional application of energy is required. Generally, when a treatment has been performed, another set of CT or MRI scan needs to be performed to check the level of treatment. However, since the sensors 255 are capable of detecting or otherwise sensing the fluorescent color previously applied, the level of treatment may be monitored as the treatment is being performed and such data may be presented to a user for viewing.

It is contemplated that any of the sensors 255 described above may also, or alternatively, be included on an extended working channel 12 or any other component of the present disclosure.

Fig. 4 illustrates an extended working channel 12 including an ultrasound transducer 401 and at least one sensor 455 located at a distal portion of the extended working channel 12. One skilled in the art will recognize that the ultrasound transducer 401 and sensor 455 may be a component of microwave ablation device 60, and therefore, will only be described with respect to extended working channel 12 for brevity. The ultrasound transducer 401 transmits ultrasound waves and sensor 455 receives reflected ultrasound waves. Generally, ultrasound waves penetrate tissue based on the frequency of the ultrasound waves. For example, 1 megahertz (MHz) ultrasound waves penetrate to a depth of 2 cm to 5 cm and 3 MHz ultrasound waves penetrate to a depth of 1.5 cm. Thus, ultrasound waves are suitable for imaging bronchial trees. In an aspect, the ultrasound transducer 401 is a radial ultrasound transducer.

Generally, ultrasound waves are reflected at a boundary where density changes or at the interface between tissues. While the ultrasound transducer 401 is navigating the luminal network of the lung, the ultrasound waves are reflected from the inside wall of a bronchial tree, from the outside wall of the bronchial tree, and from a diseased portion or cancerous portion located at the outside wall of the bronchial tree and provide finite details of the lung structure and the tissue patency that could not otherwise be revealed using non-invasive imaging means.

The reflected ultrasound waves sensed by sensors 455 have information such as amplitude and a delayed time between transmission of the ultrasound waves and reception of the reflected ultrasound waves. Since the ultrasound waves travels differently and attenuates amplitudes differently in accordance with the density of tissue, the amplitude and the delayed time may be used to identify a type of tissue, a density of the tissue, and/or a size of the tissue (for example, before and after application of energy to the tissue). Since the density of abnormal tissues (e.g., diseased or cancerous cells) are different from the normal lung tissue, the reflected ultrasound waves may be used to identify the diseased or cancerous cells from normal cells and the size and/or thickness of the diseased or cancerous cells.

The computing device 125 analyzes the reflected ultrasound waves sensed by sensors 455 and generates visual images which has a higher resolution than that of the 3D model or the CT scan images. The generated visual images may be augmented by and integrated with the 3D model of the lung or 2D images such as the CT scan images and displayed on a display of the computing device 125 for a clinician to view. Additionally or alternatively, the computing device 125 may utilize the reflected ultrasound waves (or any other sensed signals described herein) to determine a treatment plan including the amount of time to apply energy and the type of energy to apply, or other ablation/treatment levels or doses using the sensed signals.

In embodiments, when a treatment is performed to treat an abnormal tissue located at the outside wall of a bronchial tree, generally, the size of the abnormal tissue shrinks and density of the abnormal tissue changes to the density of the normal lung tissue. Traditionally, when a treatment is performed, another CT scan is performed to obtain another set of CT images to check the size of the diseased or cancerous cells so that clinicians may determine whether the treatment is complete or another one is to be made. Since the sensors 455 are able to check the size and the density of the abnormal tissue, the level of treatment may also be monitored and the ablation zones assessed during application of treatment, such as microwave energy, and after completion of treatment without the need for performing another CT scan.

In embodiments, the ultrasound transducer 401, sensors 455, and the computing device 125 may monitor the size of the target 14 before, during, and after treatment (application of microwave energy). When the size of the target 14 is greater than a threshold size, another treatment may be necessary to complete the treatment. Thus, the treatment continues until the size of the target 14 is decreased under the threshold size. In this way, visualization using ultrasound waves may be utilized for monitoring the treatment.

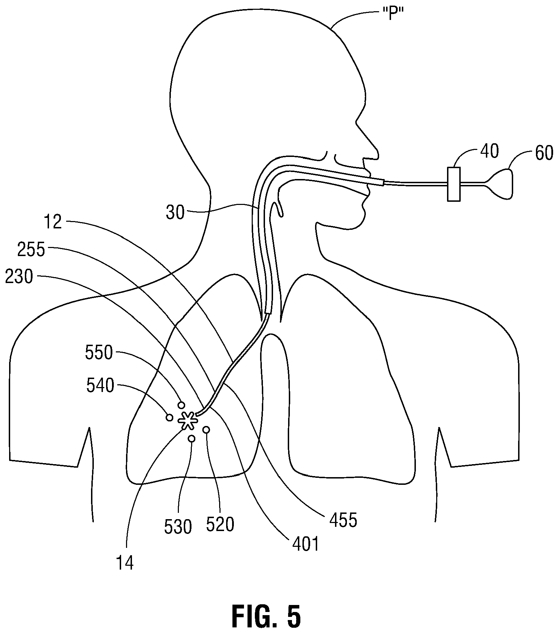

Fig. 5 depicts the microwave ablation device 60 of either Fig. 2 or Fig. 3 navigated to a region of interest within a bronchial airway of a patient "P" where a target 14 is located. The area of interest, where the target 14 is located, may include a plurality of thermal areas or regions or zones 520, 530, 540, 550. Each thermal zone 520, 530, 540, 550 may exhibit different tissue characteristics. For example, each thermal zone 520, 530, 540, 550 may have a different temperature or temperature range. One common reason for different thermal zones within tissue is that the tissue in question is of a different type or has a different composition. For example, cancerous lesions and tumors, such as target 14, typically have heightened vasculature as compared to healthy tissue. As a result, lesions and tumors, such as target 14, typically have a greater water content and blood content than surrounding tissue resulting in different emissions than healthy tissue. Similarly, bone has a very different emissions signature than muscle tissue, and these distinctions can be discerned by analyzing the emissions using radiometer 160. Of course, the tissue characteristics are not limited only to these examples. The tissue characteristics may also include, for instance, tissue volume measurements and tissue density measurements.

The differences in emissions may be useful in guiding the microwave ablation device 60 to, for example, place the microwave ablation device 60 within tissue expressing a particular emission representative of particular tissue characteristics, such as target 14. In addition, certain types of tissue may be avoided when navigating the microwave ablation device 60 to the target 14, thus avoiding the unintentional or inadvertent ablation of healthy tissue. Specifically, using the emissions data or any other data sensed by sensors 255 or sensors 455, a clinician can confirm that the microwave ablation device 60 is positioned in (or proximate) the target 14, as opposed to neighboring healthy tissue, prior to activating the microwave energy. Moreover, as described above, using the emissions data in conjunction with the display on computing device 125, a clinician may visualize and confirm the ablation zone created by the microwave antenna 230 to monitor the ablation zone and ensure that the entire target 14 is ablated without damaging neighboring healthy tissue. Even further, the emissions data may also be used to determine a treatment plan including the amount of time to apply energy and the type of energy to apply, or other ablation/treatment levels or doses.

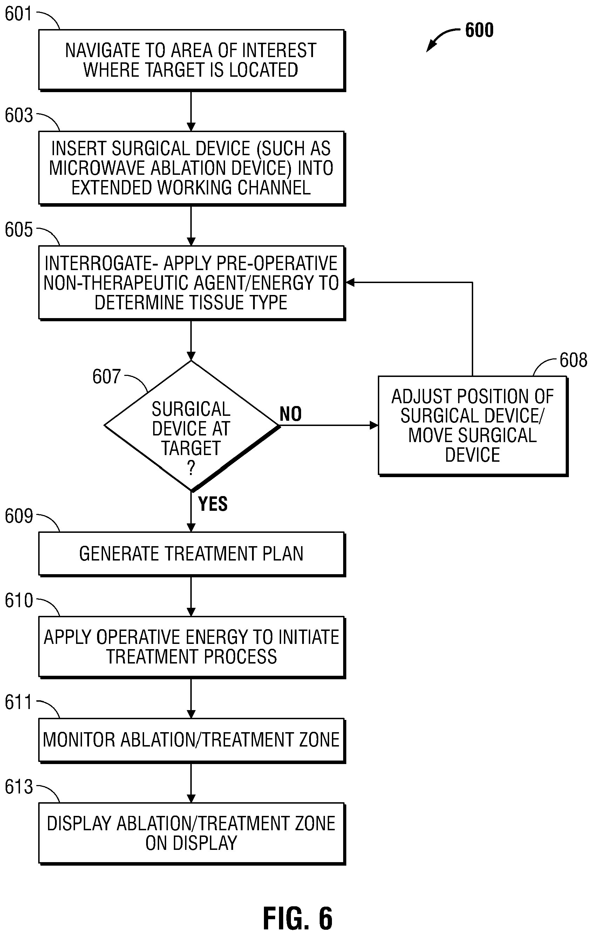

As the microwave antenna 230 approaches a target 14, the emissions are sensed prior to entry and application of energy. The sensors 255 may continuously gather data, or alternatively microwave ablation device 60 may generate an interrogating signal to which a response is received from the tissue, as described in further detail below with reference to Fig. 6.

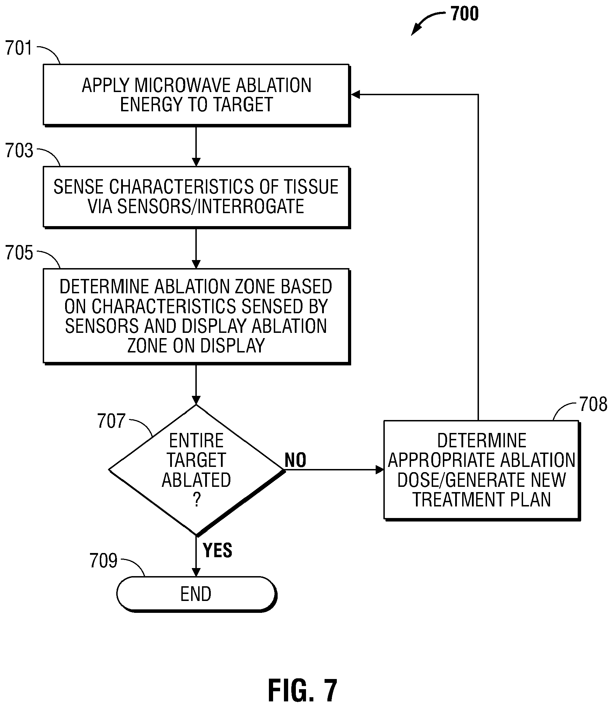

Having described the components of system 100, depicted in Figs. 1-4, the following description of Fig. 6 provides an exemplary workflow of using the components of system 100 to confirm placement of a surgical device (such as microwave ablation device 60) proximate a target 14 and Fig. 7 provides an exemplary workflow of using the components of system 100 to monitor treatment of the target 14 during and/or after application of microwave energy. Particularly, Fig. 6 illustrates a method for confirming placement of a microwave ablation device 60 relative to a target 14, generally described as method 600, and Fig. 7 illustrates a method for monitoring the ablation zone created by the microwave ablation device 60, and is generally described as method 700. Although the methods illustrated and described herein are illustrated and described as being in a particular order and requiring particular steps, any of the methods may include some or all of the steps and may be implemented in any order not specifically described.

Method 600 begins in 601 where an extended working channel 12 is navigated to an area of interest where a target 14 is located. As described above, the extended working channel 12 is navigated to the target 14 using suitable navigation software. Specifically, a previously developed navigation plan is imported into computing device 125, or generated by computing device 125, and the plan is registered with the patient's "P" location enabling a clinician to follow the plan within the patient's "P" bronchial tree (BT) with extended working channel 12 and locatable guide 32. A clinician follows the plan by advancing the bronchoscope 30, and once the bronchoscope 30 is wedged, advancing the extended working channel 12 of the catheter guide assembly 40 through the working channel of the bronchoscope 30 to the target 14. The location of the distal end of the extended working channel 12, where the locatable guide 32 is located, is monitored by the tracking system 50 as it is advanced through the BT. Further details regarding the navigation are described in U.S. Patent No. 7,233,820.

After navigating the extended working channel 12 proximate the target 14, in 603 the surgical device is inserted into extended working channel 12. When a locatable guide 32 is used, in 603 the locatable guide 32 is removed from the extended working channel 12 and the microwave ablation device 60 (or other surgical device) is inserted into the extended working channel 12 to access the target 14. Once the microwave ablation device 60 is navigated to the region of interest where the target 14 is located, in 605 the interrogation begins. In particular, in 605 an interrogation is performed where a pre-operative, non-therapeutic, energy or agent is applied to determine the type of tissue that is proximate the microwave ablation device 60. As described above, either or both of the extended working channel 12 or the microwave ablation device 60 may include sensors, such as sensors 255 (Figs. 2 and 3), and/or an ultrasound transducer 401 used in conjunction with sensors 455 (Fig. 4). The sensors 255 and/or sensors 455 may be used to sense ultrasound signals, temperature, impedance, water content, fluorescence, and reflectometry of the of the tissue proximate the microwave ablation device 60. The sensed signals are used to determine the type of tissue (healthy tissue, lung lesion, target 14, etc.) that is located in proximity to the microwave ablation device 60.

In 607, it is determined whether the microwave ablation device 60 is positioned proximate the target 14, as opposed to being positioned proximate tissue other than the target 14 (such as healthy tissue). If it is determined that the microwave ablation device 60 is not positioned proximate the target 14 (no in 607), then method 600 proceeds to 608. Alternatively, if it is determined that the microwave ablation device 60 is positioned proximate the target 14 (yes in 607), then method 600 proceeds to 609. In 608, computing device 125 may prompt the clinician (via a display or alarm means) that the microwave ablation device 60 is positioned proximate tissue that is not the target 14, so that the microwave ablation device 60 may be repositioned. Step 608 enables a clinician to verify the position of the surgical device (such as microwave ablation device 60) relative to the target 14 and make adjustments to the position of the surgical device relative to the target 14 before performing a surgical procedure (i.e., activation of microwave energy). After repositioning in 608, method 600 reverts to 605 to repeat the location confirmation process of 605 and 607, until the microwave ablation device 60 is positioned proximate the target 14.

Once the microwave ablation device 60 is positioned proximate the target 14 (yes in 607), a treatment plan is developed in step 609. The treatment plan considers the size and the shape of the target 14, identified in the CT image. In addition, the treatment plan considers the sensed signals from step 605, which were used to identify the type of tissue, to output a treatment plan including one or more or the amount of time to apply energy and the type of energy to apply, cyclical frequency of the application of energy and other ablation/treatment levels or doses in order to achieve the desired ablation/treatment zone.

In step 610 the operative energy (such as microwave energy) is applied. Once the operative energy is applied to the target 14 in 610, the ablation/treatment zone is continuously monitored by system 100 to determine the ablation/treatment zone created in 611. As described above, in 613, the ablation/treatment zone may be displayed on a display of computing device 125 for a clinician to monitor the amount of the target 14 that has been ablated. One specific embodiment of step 611 is described in method 700 of Fig. 7 below.

Following treatment of the target 14 and continuous monitoring, a clinician can confirm that a suitable ablation zone has been formed around the target 14 and determine whether additional application of energy is necessary. These steps of treating and monitoring may be repeated iteratively until a determination is made that the target 14 has been successfully ablated. Moreover, the methodology described above using the sensing techniques described above (radiometry, temperature, impedance, ultrasound, staining, etc.) to both confirm placement of the microwave ablation device 60 and monitoring of the ablation zone to assist in determining treatment cessation points may be used in conjunction with imaging modalities (such as fluoroscopic imaging) to confirm the extent of treatment and determine whether additional application of energy is necessary.

Specifically, one embodiment of such a method is illustrated in Fig. 7 and described generally as method 700. Method 700 begins at 701 where microwave ablation energy is applied to a target 14 via a microwave ablation device 60. In step 703, the characteristics of the target tissue are sensed. As described above, the characteristics may include any or a combination of received ultrasound signals, temperature, impedance, water content, and reflectometry of the tissue proximate the microwave ablation device 60 sensed via sensors 255 or sensors 455.

In step 705, the ablation zone is determined based on the characteristics sensed in step 703, and the determined ablation zone is displayed. In step 707, a determination is made as to whether the entire target 14 has been ablated. As described above, a clinician may view the ablation zone on a display of computing device 125 and make a determination as to whether further ablation or application of energy is needed. Additionally, or alternatively, computing device 125 may determine whether the ablation zone exceeds a predetermined threshold value. If the ablation zone does not exceed the predetermined threshold value (no in 707), then method 700 proceeds to step 708 and reverts to step 701 where additional microwave energy is applied. Particularly, in step 708, the feedback received from sensors 255 and/or 455 in step 703 are used to determine an appropriate ablation dose (including but not limited to power levels, or duration of application of energy) to utilize in step 701. The determination of the appropriate ablation dose in step 708 may assist in ensuring that the entire target tissue will be ablated.

From the foregoing and with reference to the various figure drawings, those skilled in the art will appreciate that certain modifications can also be made to the present disclosure without departing from the scope of the same. For example, one or modifications may be made in the way of device delivery and placement; device cooling and antenna buffering; and sensor feedback.

As can be appreciated a surgical device such as a biopsy tool or an energy device, such as a microwave ablation catheter, that is positionable through one or more branched luminal networks of a patient to treat tissue may prove useful in the surgical arena and the present disclosure is directed to such apparatus, systems, and methods. Access to luminal networks may be percutaneous or through natural orifice. In the case of natural orifice, an endobronchial approach may be particularly useful in the treatment of lung disease. Targets, navigation, access and treatment may be planned pre-procedurally using a combination of imaging and/or planning software. In accordance with these aspects of the present disclosure, the planning software may offer custom guidance using pre-procedure images. Navigation of the luminal network may be accomplished using image-guidance. These image-guidance systems may be separate or integrated with the energy device or a separate access tool and may include MRI, CT, fluoroscopy, ultrasound, electrical impedance tomography, optical, and/or device tracking systems. Methodologies for locating the access tool include EM, IR, echolocation, optical, and others. Tracking systems may be integrated to an imaging device, where tracking is done in virtual space or fused with preoperative or live images. In some cases the treatment target may be directly accessed from within the lumen, such as for the treatment of the endobronchial wall for COPD, Asthma, lung cancer, etc. In other cases, the energy device and/or an additional access tool may be required to pierce the lumen and extend into other tissues to reach the target, such as for the treatment of disease within the parenchyma. Final localization and confirmation of energy device placement may be performed with imaging and/or navigational guidance using the modalities described below. The energy device has the ability to deliver an energy field for treatment (including but not limited to electromagnetic fields).

The invention is set out in the appended claims.

## Claims

1. A system for monitoring a surgical procedure, the system comprising:
a catheter guidance assembly (40) including an extended working channel (12) navigable to a target;
a surgical device (60) configured to be positioned through the extended working channel to access the target;
at least one sensor (255) disposed on at least one of the extended working channel or the surgical device, the at least one sensor configured to sense a characteristic of tissue proximate at least one of the extended working channel or the surgical device; and
a computing device (125) operably coupled to the at least one sensor, the computing device configured to receive the characteristic and determine whether the tissue characteristic is indicative of the target;
wherein the computing device is further configured to display a treatment zone of the target and determine whether the target has been treated based on the sensed characteristic of tissue.

2. The system according to claim 1, wherein the extended working channel further comprises an ultrasound transducer and an ultrasound sensor disposed on a distal portion of the extended working channel, the ultrasound transducer configured to generate ultrasound waves and the ultrasound sensor configured to receive ultrasound waves reflected from tissue.

3. The system according to claim 2, wherein the computing device is further configured to display a model of a luminal network, process the sensed ultrasound waves reflected from tissue and integrate the sensed ultrasound waves reflected from tissue with the model of a luminal network.

4. The system according to claim 1, 2 or 3, wherein the at least one sensor is at least one light receptor configured to sense light reflected from tissue proximate the surgical device, and the computing device is configured to convert the reflected light into light based data and identify a type or density of tissue, identify the presence of one or more blood vessels, generate visible images, or integrate the light based data with a model for display.

5. The system according to claim 1, 2, 3 or 4, further comprising a radiometer operably coupled to the at least one sensor, the radiometer configured to measure emissions from a thermal field created when the surgical device delivers microwave energy, the thermal field providing in-situ quantitative information of a material in the thermal field, optionally wherein the radiometer is disposed within a handle of the surgical device or wherein the radiometer is disposed external to a handle of the surgical device.

6. The system according to claim 5, wherein the quantitative information is at least one of thermal conductivity, specific heat, density, or blood perfusion, optionally wherein the quantitative information is displayed on a display of the computing device.

7. The system according to any preceding claim, wherein the at least one sensor is at least one hydration sensor configured to detect water levels of tissue proximate the at least one hydration sensor.

8. The system according to any preceding claim, wherein the computing device is further configured to identify tissue density based on the characteristic of tissue.

9. The system according to any preceding claim, wherein the surgical device is a microwave ablation device configured to deliver microwave energy to the target.

10. The system according to any preceding claim, further comprising a microwave generator configured to generate microwave energy and deliver the microwave energy to the surgical device.

11. A system according to any preceding claim for monitoring a surgical procedure, wherein the computing device is configured to:
display preplanned navigation plan enabling navigation of an extended working channel and a surgical device to a target;;
sense a response of applied non-therapeutic amount of energy via the at least one sensor;
determine whether the tissue proximate the surgical device is the target by analyzing the sensed response;
generate a treatment plan when it is determined that the tissue proximate the surgical device is the target;
enable the application of therapeutic energy in accordance with the treatment plan; and
monitor the application of the applied therapeutic energy.

12. The system according to claim 11, wherein the computing device is configured to display a representation of an ablation zone created by the therapeutic energy on a graphical user interface,
optionally wherein the computing device is configured to :
determine whether the ablation zone exceeds a threshold value; and
apply additional therapeutic energy when it is determined that the ablation zone does not exceed the threshold value.

13. The system according to claim 11 or 12, wherein the computing device is configured to:
combine at least a portion of the sensed response with a representation of a lung into a combined image; and
display the combined image on a graphical user interface.

14. A non-transitory computer readable storage medium including instructions that when executed by the computing device of claim 1 causes the computing device to:
display preplanned navigation plan enabling navigation of an extended working channel and a surgical device to a target;
sense a response of an applied non-therapeutic amount of energy via a sensor;
determine whether the tissue proximate the surgical device is the target by analyzing the sensed response;
enable application of a therapeutic energy when it is determined that the tissue proximate the surgical device is the target; and
monitor the application of the applied therapeutic energy;
display a treatment zone of the target; and
determine whether the target has been treated based on the sensed characteristic of tissue.

## Patentansprüche

1. System zum Überwachen eines chirurgischen Verfahrens, wobei das System Folgendes umfasst:
eine Katheterführungsanordnung (40) mit einem erweiterten Arbeitskanal (12), der zu einem Ziel navigierbar ist;
eine chirurgische Vorrichtung (60), die ausgestaltet ist, um durch den erweiterten Arbeitskanal positioniert zu werden, um auf das Ziel zuzugreifen;
mindestens einen Sensor (255), der auf mindestens einem der erweiterten Arbeitskanäle oder der chirurgischen Vorrichtung angeordnet ist, wobei der mindestens eine Sensor ausgestaltet ist, um eine Eigenschaft von Gewebe in der Nähe von mindestens einem von dem erweiterten Arbeitskanal oder der chirurgischen Vorrichtung zu erfassen; und
eine Rechenvorrichtung (125), die funktionsfähig mit dem mindestens einen Sensor gekoppelt ist, wobei die Rechenvorrichtung ausgestaltet ist, um die Eigenschaft zu empfangen und zu bestimmen, ob die Gewebecharakteristik für das Ziel indikativ ist;
wobei die Rechenvorrichtung ferner ausgestaltet ist, um eine Behandlungszone des Ziels anzuzeigen und zu bestimmen, ob das Ziel auf Grundlage der erfassten Gewebeeigenschaft behandelt wurde.

2. System nach Anspruch 1, wobei der erweiterte Arbeitskanal ferner einen Ultraschallwandler und einen Ultraschallsensor umfasst, die an einem distalen Abschnitt des erweiterten Arbeitskanals angeordnet sind, wobei der Ultraschallwandler ausgestaltet ist, um Ultraschallwellen zu erzeugen, und der Ultraschallsensor ausgestaltet ist, um vom Gewebe reflektierte Ultraschallwellen zu empfangen.

3. System nach Anspruch 2, wobei die Rechenvorrichtung ferner ausgestaltet ist, um ein Modell eines luminalen Netzwerks anzuzeigen, die erfassten Ultraschallwellen,die vom Gewebe reflektiert werden, zu verarbeiten, und die erfassten Ultraschallwellen, die vom Gewebe reflektiert werden, mit dem Modell eines luminalen Netzwerks zu integrieren.

4. System nach Anspruch 1, 2 oder 3, wobei der mindestens eine Sensor mindestens ein Lichtempfänger ist, der ausgestaltet ist, um Licht zu erfassen, das vom Gewebe in der Nähe der chirurgischen Vorrichtung reflektiert wird, und wobei die Rechenvorrichtung ausgestaltet ist, um das reflektierte Licht in lichtbasierte Daten umzuwandeln und eine Art oder Dichte von Gewebe zu identifizieren, das Vorhandensein eines oder mehrerer Blutgefäße zu identifizieren, sichtbare Bilder zu erzeugen oder die lichtbasierten Daten mit einem Modell zur Anzeige zu integrieren.

5. System nach Anspruch 1, 2, 3 oder 4, ferner umfassend ein Radiometer, das funktionsfähig mit dem mindestens einen Sensor gekoppelt ist, wobei das Radiometer ausgestaltet ist, um Emissionen aus einem Wärmefeld zu messen, das erzeugt wird, wenn die chirurgische Vorrichtung Mikrowellenenergie liefert, wobei das Wärmefeld *in-situ* quantitative Informationen über ein Material im Wärmefeld liefert, optional wobei das Radiometer in einem Griff der chirurgischen Vorrichtung angeordnet ist oder wobei das Radiometer außerhalb eines Griffs der chirurgischen Vorrichtung angeordnet ist.

6. System nach Anspruch 5, wobei die quantitative Information mindestens eines von Wärmeleitfähigkeit, spezifischer Wärme, Dichte oder Blutdurchblutung ist, optional wobei die quantitative Information auf einer Anzeige der Rechenvorrichtung angezeigt wird.

7. System nach einem vorhergehenden Anspruch, wobei der mindestens eine Sensor mindestens ein Hydrationssensor ist, der ausgestaltet ist, um Wasserstände von Gewebe in der Nähe des mindestens einen Hydrationssensors zu erfassen.

8. System nach einem vorhergehenden Anspruch, wobei die Rechenvorrichtung ferner ausgestaltet ist, um die Gewebedichte auf Grundlage der Gewebeeigenschaft zu identifizieren.

9. System nach einem vorhergehenden Anspruch, wobei die chirurgische Vorrichtung eine Mikrowellenablationsvorrichtung ist, die ausgestaltet ist, um Mikrowellenenergie an das Ziel zu liefern.

10. System nach einem vorhergehenden Anspruch, ferner umfassend einen Mikrowellengenerator, der ausgestaltet ist, um Mikrowellenenergie zu erzeugen und die Mikrowellenenergie an die chirurgische Vorrichtung zu liefern.

11. System nach einem vorhergehenden Anspruch zum Überwachen eines chirurgischen Verfahrens, wobei die Rechenvorrichtung für Folgendes ausgestaltet ist:
Anzeigen eines vorgeplanten Navigationsplans, der die Navigation eines erweiterten Arbeitskanals und einer chirurgischen Vorrichtung zu einem Ziel ermöglicht;
Erfassen einer Reaktion der angewandten nicht-therapeutischen Energiemenge über den mindestens einen Sensor;
Bestimmen durch Analysieren der erfassten Reaktion, ob das Gewebe in der Nähe der chirurgischen Vorrichtung das Ziel ist;
Erzeugen eines Behandlungsplans, wenn bestimmt wird, dass das Gewebe in der Nähe der chirurgischen Vorrichtung das Ziel ist;
Ermöglichen der Anwendung therapeutischer Energie in Übereinstimmung mit dem Behandlungsplan; und
Überwachen der Anwendung der angewandten therapeutischen Energie.

12. System nach Anspruch 11, wobei die Rechenvorrichtung ausgestaltet ist, um eine Darstellung einer Ablationszone, die durch die therapeutische Energie erzeugt wird, auf einer grafischen Benutzeroberfläche anzuzeigen, optional wobei die Rechenvorrichtung für Folgendes ausgestaltet ist:
Bestimmen, ob die Ablationszone einen Schwellenwert überschreitet; und
Anwenden von zusätzlicher therapeutischer Energie, wenn festgestellt wird, dass die Ablationszone den Schwellenwert nicht überschreitet.

13. System nach Anspruch 11 oder 12, wobei die Rechenvorrichtung für Folgendes ausgestaltet ist:
Kombinieren mindestens eines Teil der erfassten Reaktion mit einer Darstellung einer Lunge in einem kombinierten Bild; und
Anzeigen des kombinierten Bilds auf einer grafischen Benutzeroberfläche.

14. Nichtflüchtiges, computerlesbares Speichermedium mit Anweisungen, die bei Ausführung durch die Rechenvorrichtung nach Anspruch 1 die Rechenvorrichtung zu Folgendem veranlassen:
Anzeigen eines vorgeplanten Navigationsplans, der die Navigation eines erweiterten Arbeitskanals und einer chirurgischen Vorrichtung zu einem Ziel ermöglicht;
Erfassen einer Reaktion einer angewandten nicht-therapeutischen Energiemenge über einen Sensor;
Bestimmen durch Analysieren der erfassten Reaktion, ob das Gewebe in der Nähe der chirurgischen Vorrichtung das Ziel ist;
Ermöglichen der Anwendung einer therapeutischen Energie, wenn bestimmt wird, dass das Gewebe in der Nähe der chirurgischen Vorrichtung das Ziel ist; und
Überwachen der Anwendung der angewandten therapeutischen Energie;
Anzeigen einer Behandlungszone des Ziels; und
Bestimmen, ob das Ziel auf der Grundlage der erfassten Gewebecharakteristik behandelt wurde.

## Revendications

1. Système de surveillance d'une intervention chirurgicale, le système comprenant :
un ensemble de guidage de cathéter (40) comprenant un canal de travail étendu (12) navigable jusqu'à une cible ;
un dispositif chirurgical (60) conçu pour être positionné à travers le canal de travail étendu afin d'accéder à la cible ;
au moins un capteur (255) disposé sur le canal de travail étendu et/ou le dispositif chirurgical, l'au moins un capteur étant conçu pour détecter une caractéristique de tissu à proximité du canal de travail étendu et/ou du dispositif chirurgical ; et
un dispositif informatique (125) accouplé de manière fonctionnelle à l'au moins un capteur, le dispositif informatique étant conçu pour recevoir la caractéristique et déterminer si la caractéristique du tissu indique la cible ;
le dispositif informatique étant en outre conçu pour afficher une zone de traitement de la cible et déterminer si la cible a été traitée sur la base de la caractéristique détectée du tissu.

2. Système selon la revendication 1, dans lequel le canal de travail étendu comprend en outre un transducteur à ultrasons et un capteur à ultrasons disposés sur une partie distale du canal de travail étendu, le transducteur à ultrasons étant conçu pour générer des ondes ultrasonores et le capteur à ultrasons étant conçu pour recevoir des ondes ultrasonores réfléchies par le tissu.

3. Système selon la revendication 2, dans lequel le dispositif informatique est en outre conçu pour afficher un modèle d'un réseau luminal, traiter les ondes ultrasonores détectées réfléchies par le tissu et pour intégrer les ondes ultrasonores détectées réfléchies par le tissu à l'aide du modèle d'un réseau luminal.

4. Système selon la revendication 1, 2 ou 3, dans lequel l'au moins un capteur est au moins un récepteur de lumière conçu pour détecter la lumière réfléchie par le tissu à proximité du dispositif chirurgical, et le dispositif informatique est conçu pour convertir la lumière réfléchie en données basées sur la lumière et pour identifier un type ou une densité de tissu, identifier la présence d'un ou de plusieurs vaisseaux sanguins, générer des images visibles, ou pour intégrer les données basées sur la lumière dans un modèle destiné à être affiché.

5. Système selon la revendication 1, 2, 3 ou 4, comprenant en outre un radiomètre accouplé de manière fonctionnelle à l'au moins un capteur, le radiomètre étant conçu pour mesurer les émissions à partir d'un champ thermique créé lorsque le dispositif chirurgical fournit de l'énergie à micro-ondes, le champ thermique fournissant des informations quantitatives in situ d'un matériau dans le champ thermique, éventuellement dans lequel le radiomètre est disposé à l'intérieur d'une poignée du dispositif chirurgical, ou dans lequel le radiomètre est disposé à l'extérieur d'une poignée du dispositif chirurgical.

6. Système selon la revendication 5, dans lequel les informations quantitatives sont la conductivité thermique et/ou la chaleur spécifique et/ou la densité et/ou la perfusion sanguine, éventuellement dans lequel les informations quantitatives sont affichées sur un écran du dispositif informatique.

7. Système selon l'une des revendications précédentes, dans lequel l'au moins un capteur est au moins un capteur d'hydratation conçu pour détecter des niveaux d'eau du tissu à proximité de l'au moins un capteur d'hydratation.

8. Système selon l'une des revendications précédentes, dans lequel le dispositif informatique est en outre conçu pour identifier la densité de tissu sur la base de la caractéristique du tissu.

9. Système selon l'une des revendications précédentes, dans lequel le dispositif chirurgical est un dispositif d'ablation à micro-ondes conçu pour fournir de l'énergie à micro-ondes à la cible.

10. Système selon l'une des revendications précédentes, comprenant en outre un générateur de micro-ondes conçu pour générer de l'énergie à micro-ondes et pour fournir de l'énergie à micro-ondes au dispositif chirurgical.

11. Système selon l'une des revendications précédentes, permettant de surveiller une intervention chirurgicale, dans lequel le dispositif informatique est conçu pour :
afficher un plan de navigation pré-planifié permettant la navigation d'un canal de travail étendu et d'un dispositif chirurgical vers une cible ;
détecter une réponse de quantité d'énergie non thérapeutique appliquée par l'intermédiaire de l'au moins un capteur ;
déterminer si le tissu à proximité du dispositif chirurgical est la cible en analysant la réponse détectée ;
générer un plan de traitement lorsqu'il est déterminé que le tissu se trouvant à proximité du dispositif chirurgical est la cible ;
permettre l'application d'énergie thérapeutique conformément au plan de traitement ; et pour
surveiller l'application de l'énergie thérapeutique appliquée.

12. Système selon la revendication 11, dans lequel le dispositif informatique est conçu pour afficher une représentation d'une zone d'ablation créée par l'énergie thérapeutique sur une interface d'utilisateur graphique, éventuellement dans lequel le dispositif informatique est conçu pour :
déterminer si la zone d'ablation dépasse une valeur seuil ; et pour
appliquer de l'énergie thérapeutique supplémentaire lorsqu'il est déterminé que la zone d'ablation ne dépasse pas la valeur seuil.

13. Système selon la revendication 11 ou 12, dans lequel le dispositif informatique est conçu pour :
combiner au moins une partie de la réponse détectée avec une représentation d'un poumon en une image combinée ; et pour
afficher l'image combinée sur une interface d'utilisateur graphique.

14. Support de stockage non transitoire lisible par ordinateur comprenant des instructions qui, lorsqu'elles sont exécutées par le dispositif informatique selon la revendication 1, amènent le dispositif informatique à :
afficher un plan de navigation pré-planifié permettant la navigation d'un canal de travail étendu et d'un dispositif chirurgical vers une cible ;
détecter une réponse d'une quantité d'énergie non thérapeutique appliquée par l'intermédiaire d'un capteur ;
déterminer si le tissu à proximité du dispositif chirurgical est la cible en analysant la réponse détectée ;
permettre l'application d'une énergie thérapeutique lorsqu'il est déterminé que le tissu se trouvant à proximité du dispositif chirurgical est la cible ;
surveiller l'application de l'énergie thérapeutique appliquée ;
afficher une zone de traitement de la cible ; et à
déterminer si la cible a été traitée sur la base de la caractéristique détectée du tissu.
